# EUROPEAN PATENT SPECIFICATION

(11) **EP 4 028 066 B1**
(45) Date of publication and mention of the grant of the patent: **15.01.2025**
(21) Application number: 20862937.8
(22) Date of filing: 03.09.2020
(51) Int. Cl.: A61L 24/02, A61L 24/00, A61L 26/00, A61B 17/12, A61B 17/00

(54) **SILICA FIBER HEMOSTATIC DEVICES AND METHODS**
HÄMOSTATISCHE PRODUKTE AUS SILICAFASERN UND VERFAHREN
DISPOSITIFS ET PROCÉDÉS HÉMOSTATIQUES À BASE DE FIBRES DE SILICE

(30) Priority: 10.09.2019 US 201962898148 P; 31.03.2020 US 202063002475 P
(43) Date of publication of application: 20.07.2022
(73) Proprietor: AMERICAN NANO, LLC, Clemmons, NC 27012 (US)
(72) Inventor: DELLINGER, Mitch, Clemmons, NC 27012 (US)
(74) Representative: Maiwald GmbH
(86) International application number: PCT/US2020/049111
(87) International publication number: WO 2021/050344

(56) References cited:
- US-A1- 2003 032 919
- US-A1- 2007 160 653
- US-A1- 2011 077 682
- US-A1- 2011 077 682
- US-A1- 2013 237 898
- US-B1- 10 111 783
- US-B1- 10 111 783
- DELONG HUGH: "Silica Nanofiber Combat Hemostat", October 2008 (2008-10-01), XP093041721, Retrieved from the Internet <URL:https://www.researchgate.net/publication/235068577_Silica_Nanofiber_Combat_Hemostat_SINCH> [retrieved on 20230424]

## Description

### Related Applications

This application is claims the benefit of and priority to U.S. Provisional Patent Application No. 62/898,148, filed September 10, 2019, and U.S. Provisional Patent Application No. 63/002,475, filed March 31, 2020.

### Technical Field

In various embodiments, the present invention relates to hemostatic devices and methods utilizing silica fibers.

### Background

Excessive bleeding or hemorrhaging is a significant cause of mortality after traumatic injury and battlefield injuries. Excess bleeding is also a complication during surgical procedures. Hemostatic products and processes are intended to assist in the rapid initiation of a hemostatic plug formed through platelet activation, aggregation, adhesion and gross clot formation at a tissue target site.

While a wide variety of hemostatic products have been made from different base materials, such as collagen, gelatin, cellulose, chitosan, and fibrin, these materials fail for many applications, such as internal hemorrhage where direct pressure to the wound is difficult or impossible.

Delong Hugh "Silica Nanofiber Combat Hemostat", October 2008, URL:https:// www.researchgate.net/publication/235068577_Silica_Nanofiber_Combat_Hemostat_SINCH discloses the hemostatic capabilities of a combination of silica nanofibers with glass microspheres.

The present invention in various aspects and embodiments provides hemostats for rapid control of hemorrhages, as well as methods and kits for their use.

### Summary

The scope of this invention is defined by the claims. Any "embodiment" or "example" which is disclosed in the description but is not covered by the claims should be considered as presented for illustrative purpose only. Embodiments in the description relating to methods of treatment are not covered by the claims.

The present invention provides a electrospun silica fiber composition for use in treating hemorrhage according to claim 1. The electrospun silica fibers when applied to a site of hemorrhage, are able to quickly stop the hemorrhage. Thus, the invention provides hemostatic electrospun fibers beneficial for treating subjects experiencing severe injuries, including for emergency treatment at the site of the injury. The invention further is useful in the surgical setting for management of bleeds during surgery. In various embodiments, the invention finds use for treating external wounds and injuries of varying severity. Also disclosed but not according to the claimed invention are delivery devices for the hemostatic device and material.

The electrospun silica material is able to hold many times its weight in water, (e.g., typically more than 50 times its weight in water), which allows the material to absorb a large amount of blood, while being impenetrable by blood cells and quickly forming a physical barrier. In various embodiments, the silica fiber composition enhances coagulation pathways. The silica fiber material may be applied as a flocculent material, or alternatively, applied as a powder or dust, gel, paste, or as an additive to a flowable base material.

In some embodiments, the silica fiber composition is a lightweight structure consisting essentially of silica fiber (e.g., silicon dioxide nanofiber). In various embodiments, the fiber composition consists essentially of SiO₂, i.e., contains only SiO₂ and unintentional impurities, and, in some embodiments, species and/or complexes resulting from the incomplete conversion of the sol to SiO₂ (e.g., water and/or chemical groups such as ethoxy groups, silanol groups, hydroxyl groups, etc.). The composition may be formed from a gelatinous material that is electrospun to form a fiber mat (e.g., a non-woven mat). For example, the composition may be prepared by electrospinning a sol-gel. An exemplary sol-gel is prepared with a silicon alkoxide reagent, such as tetraethyl ortho silicate (TEOS), alcohol solvent, and an acid catalyst. In various embodiments, the sol is transitioned for at least two days (and, in various embodiments, less than seven days) under conditions where humidity and temperature are controlled. The sol-gel is electrospun to create a silica fiber mat with superior texture and properties, which may find use as or in a hemostatic device. The fibers may have a variable diameter, such as in the range of from about 50 nm to 5 µm.

The material may be used as fiber mat portions that are folded and/or clumped to prepare a suitable mass of material for packing a hemorrhaging site, including a hemorrhage in a body cavity. In some embodiments, the silica fiber material is processed into a flocculent material that is loosely clumped, and may be created by shredding and/or clumping portions of the electrospun fiber mats. In some embodiments, the silica fiber material is provided with a material to facilitate handling and application to the wound site, such as any suitable backing material or flexible container. Alternatively, the silica fiber material may be applied as a powder or dust, gel, paste, or as an additive to a flowable base material, and such compositions may optionally comprise one or more bulking agents. Exemplary bulking agents include, but are not limited to, various synthetic and natural polymers.

In some embodiments, the fiber composition is applied directly to the site of hemorrhage by packing the site with the material, in any form, and applying pressure where and as possible. The fiber composition will absorb the blood and large amounts of fluid, although the blood cells cannot penetrate the material, and will quickly form a physical barrier for further loss of blood. In various embodiments, blood or extracellular matrix proteins will bind to the material, to aid the formation of a barrier, and which may provide additional benefits such as the reduction of pain and facilitation of healing. In some embodiments, even for severe hemorrhage (e.g., grade 3 or 4 hemorrhage), the silica fiber material can stop blood flow within about 60 seconds, or in some embodiments, within about 30 seconds, or in some embodiments within about 15 seconds. By quickly stopping a severe hemorrhage at the site of injury, the patient may more quickly and safely be transported to a surgical facility. The silica fiber material may be easily removed by a physician during surgery.

In some embodiments, the hemorrhage is bleeding during or after surgery. In some embodiments, the hemorrhage is a traumatic injury bleed, such as a non-compressible hemorrhage. In various embodiments, the hemorrhage is a result of a crushing injury, a gunshot injury or explosion injury.

Other useful applications include epistaxis and external wounds. For example, the silica fiber material may be shaped as pads or bandages, or used with various types of conventional bandage materials (e.g., cotton gauze) and/or wound dressings to promote bleeding control.

Other aspects and embodiments of the invention will be apparent from the following non-limiting examples.

In an aspect, there is disclosed a method for treating a subject for hemorrhage ( not part of the claimed invention). The method includes, consists essentially of, or consists of applying an electrospun silica fiber composition to said hemorrhage in an amount and under conditions sufficient to stop or slow the hemorrhage. The composition is prepared by electrospinning a sol. The sol is prepared with tetraethyl orthosilicate (TEOS). The sol contains 70% to 90% TEOS by weight, 8% to 25% ethanol by weight, an acid catalyst, and the balance water. The sol may contain 70% to 90% TEOS by weight, 8% to 25% ethanol by weight, 1% to 10% water by weight, and the acid catalyst. The sol may contain 75% to 85% by weight TEOS, 12% to 20% by weight ethanol, and about 2% to 5% by weight water. The sol may contain about 80% by weight TEOS, about 17% by weight ethanol, and about 3% by weight water. The acid catalyst may include, consist essentially of, or consist of HCl. The sol may contain less than about 0.1% of the acid catalyst by weight. The sol may contain from 0.02% to 0.08% of the acid catalyst by weight.

The sol may be allowed to transition for at least 2 days under conditions where humidity is within the range of about 40% to about 80%, and the temperature is within the range of 10 to 32.2 °C (50 to 90°F). The sol may be allowed to transition for at least 3 days, at least 4 days, at least 5 days, at least 6 days, or at least 7 days. The sol may be allowed to transition for 2 days to 7 days. The sol may be electrospun when the weight is at from 20% to 40% of the starting weight before ripening (transitioning). The sol may be electrospun when the production of ethylene vapor is 10% to 20% relative to the peak production of ethylene vapors during ripening (transitioning). The sol may be electrospun when the production of ethylene vapor therefrom is 10% to 40% relative to the sol before ripening (transitioning). The sol may not exposed to heat over 65.6°C (150°F) or heat over 37.8°C (100°F). Fibers of the silica fiber composition may have a variable diameter of from about 50 nm to about 5 µm. The fibers may have a variable diameter of from about 200 nm to about 1000 nm. The composition may include, consist essentially of, or consist of SiO₂. The composition may be electrospun with a thickness of from about 1/8 inch to about 1/4 inch. The composition may be electrospun with a thickness of greater than about 0.32 cm (1/8 inch), or greater than about 0.64 cm (1/4 inch).

The subject may be a mammal. The subject may be a veterinary patient, e.g., a dog, cat, or horse. The subject may be a human patient. The hemorrhage may be at least a Grade 2 hemorrhage, for example a Grade 3 or Grade 4 hemorrhage. The hemorrhage may be an arterial hemorrhage. The hemorrhage may be a venous hemorrhage. The hemorrhage may be bleeding during or after surgery. The hemorrhage may be an organ bleed. The hemorrhage may be a primary hemorrhage. The hemorrhage may be a reactionary hemorrhage. The hemorrhage may be a secondary hemorrhage. The hemorrhage may be a traumatic injury bleed. The hemorrhage may be a non-compressible hemorrhage. The hemorrhage may be a cavity bleed. The injury may be a crushing injury, a gunshot injury, or an explosion injury. The hemorrhage may be epistaxis. The hemorrhage may be external on the subject. The subject may be on therapy inhibiting the coagulation pathway. The subject may have a coagulation disorder. The subject may have hemophilia, thrombocytopenia, low platelet count, or von Willebrand disease.

The silica fiber composition may applied to the hemorrhage via a delivery device. The delivery device may include, consist essentially of, or consist of a hollow central bore, a movable plunger, and an annular outer compartment coaxial with the central bore. The central bore may have an upper end and an open lower end. The plunger may be disposed at the upper end of the central bore. The plunger may be actuatable by a user of the delivery device to urge the silica fiber composition from the open lower end of the central bore. A portion of the plunger may separate the central bore into a lower compartment and an upper compartment. The outer compartment may be fluidly connected to the upper compartment of the central bore. The outer compartment may define one or more openings for fluid connection with an area proximate the hemorrhage. When the plunger is actuated to urge the silica fiber composition from the open lower end of the central bore to the hemorrhage, fluid proximate the hemorrhage may be simultaneously aspirated into the outer compartment and thence into the upper compartment of the central bore. The central bore may define one or more openings therethrough fluidly connecting the lower compartment with the annular outer

A delivery device for application of a silica fiber composition proximate a hemorrhage is also disclosed (not part of the claimed invention). The delivery device includes, consists essentially of, or consists of a hollow central bore, a movable plunger, and an annular outer compartment coaxial with the central bore. The central bore has an upper end and an open lower end. The plunger is disposed at the upper end of the central bore. The plunger is actuatable by a user of the delivery device to urge the silica fiber composition from the open lower end of the central bore. A portion of the plunger separates the central bore into a lower compartment and an upper compartment. The outer compartment is fluidly connected to the upper compartment of the central bore. The outer compartment defines one or more openings for fluid connection with an area proximate the hemorrhage. When the plunger is actuated to urge the silica fiber composition from the open lower end of the central bore to the hemorrhage, fluid proximate the hemorrhage is simultaneously aspirated into the outer compartment and thence into the upper compartment of the central bore.

The invention may include one or more of the following in any of a variety of combinations. The central bore may define one or more openings therethrough fluidly connecting the lower compartment with the annular outer compartment. The silica fiber composition may be disposed within the lower compartment of the central bore. The silica fiber composition may include, consist essentially of, or consist of a plurality of silica fibers. The silica fiber composition may include, consist essentially of, or consist of a powder or dust.

In yet another aspect, embodiments of the disclosure ( not according to the claimed invention) feature a kit for treating a subject for hemorrhage. The kit includes, consists essentially of, or consists of an electrospun silica fiber composition and a delivery device. The disclosure may include one or more of the following in any of a variety of combinations. The delivery device may include, consist essentially of, or consist of a hollow central bore, a movable plunger, and an annular outer compartment coaxial with the central bore. The central bore may have an upper end and an open lower end. The plunger may be disposed at the upper end of the central bore. The plunger may be actuatable by a user of the delivery device to urge the silica fiber composition from the open lower end of the central bore. A portion of the plunger may separate the central bore into a lower compartment and an upper compartment. The outer compartment may be fluidly connected to the upper compartment of the central bore. The outer compartment may define one or more openings for fluid connection with an area proximate the hemorrhage. When the plunger is actuated to urge the silica fiber composition from the open lower end of the central bore to the hemorrhage, fluid proximate the hemorrhage may be simultaneously aspirated into the outer compartment and thence into the upper compartment of the central bore. The central bore may define one or more openings therethrough fluidly connecting the lower compartment with the annular outer compartment. The silica fiber composition may include, consist essentially of, or consist of an electrospun silica fiber composition. The silica fiber composition may include, consist essentially of, or consist of a plurality of silica fibers. The silica fiber composition may include, consist essentially of, or consist of a powder or dust.

These and other objects, along with advantages and features of the present invention herein disclosed, will become more apparent through reference to the following description, the accompanying drawings, and the claims. Furthermore, it is to be understood that the features of the various embodiments described herein are not mutually exclusive and may exist in various combinations and permutations. As used herein, the terms "approximately," "about," and "substantially" mean ±10%, and in some embodiments, ±5%. The term "consists essentially of" means excluding other materials that contribute to function, unless otherwise defined herein. Nonetheless, such other materials may be present, collectively or individually, in trace amounts.

### Brief Description of the Drawings

In the drawings, like reference characters generally refer to the same parts throughout the different views. Also, the drawings are not necessarily to scale, emphasis instead generally being placed upon illustrating the principles of the invention. In the following description, various embodiments of the present invention are described with reference to the following drawings, in which:
FIGS. 1A and 1B depict embodiments of the invention using a gauze bag (FIG. 1A) or gauze backing (FIG. 1B) to facilitate handling of the silica fiber material.
FIG. 2 shows an embodiment of the invention in which a silica fiber pad is shaped as a disc.
FIGS. 3A-3C depict an embodiment of a delivery device for application of a silica fiber composition to a wound site, including an end-on view (FIG. 3A), a cross-sectional view (FIG. 3B), and a cutaway interior view (FIG. 3C).
FIGS. 4A-4D are scanning electron microscopy (SEM) images of fibers spun in accordance with this disclosure. Images in FIGS. 4A-4D are at, respectively, 50, 100, 200, and 500 micron scale. As shown, the fibers are flexible, smooth, dense, and continuous (not fractured).
FIGS. 5A-5D are SEM images of fibers that were electrospun at a non-optimal time (before the sol-gel was fully ripened). Images in FIGS. 5A-5D are at, respectively, 50, 100, 200, and 500 micron scale. As shown, the fibers appear rigid, with many fractures visible, and with formation of clumps.
FIG. 6 shows an SEM image (20 micron scale is shown) of fibers spun at a non-optimal time. The fibers are rigid, with fractures clearly evident.
FIG. 7 shows a fiber mat spun with a thickness of about 0.64 cm (1/4 inch) in accordance with the disclosure. The mat has a soft, flexible texture.
FIGS. 8A and 8B compare a silica fiber mat that was electrospun when the sol-gel was transitioned in accordance with this disclosure (FIG. 8A), with a fiber mat that was spun too early, before the sol-gel was optimally ripened (FIG. 8B). The material in FIG. 8A has a soft texture, is very flexible, and can be spun at a thickness that is easily handled for application of fiber layers to a wound. The material in FIG. 8B is brittle, inflexible, and layers of fiber cannot be easily separated for covering the surface area of a wound.
FIGS. 9A and 9B are histology images, utilizing hematoxylin and eosin staining (FIG. 9A) and Martius-scarlet-blue (MSB) trichrome fibrin staining (FIG. 9B), of an arterial injury site of a porcine model treated with a silica fiber composition, in accordance with embodiments of the invention, during a hemostasis study.

### Detailed Description

The present invention provides compositions and methods for treating hemorrhage (bleeding) in a subject. In various aspects, the invention provides compositions comprising electrospun silica fibers, that when applied to a site of hemorrhage, are able to quickly stop the hemorrhage. Thus, the invention provides hemostatic devices and methods beneficial for treating subjects experiencing severe injuries, including for emergency treatment at the site of the injury. The invention further is useful in the surgical setting for management of bleeds during surgery. In various embodiments, the invention finds use for treating external wounds and injuries of varying severity.

The electrospun silica material is able to hold many times its weight in water, (e.g., typically more than 50 times its weight in water), which allows the material to absorb a large amount of blood, while being impenetrable by blood cells and quickly forming a physical barrier. In some embodiments, the silica material will bind extracellular matrix proteins and/or serum proteins, aiding in the formation of a barrier. In various embodiments, the silica fiber composition enhances coagulation. In the case of cavity wounds, the hemorrhaging site and/or cavity may be filled or packed with the silica fiber material. In some embodiments, the silica fiber material is applied more discriminately to the site of hemorrhage. The silica fiber material may be applied as a flocculent material, or alternatively, applied as powder, dust, gel, paste, or as an additive to a flowable base material.

In some embodiments, the silica fiber composition is a lightweight structure consisting essentially of silica fiber (e.g., silicon dioxide nanofiber). The composition may be formed from a gelatinous material that is electrospun to form a fiber mat (e.g., a non-woven mat). For example, the composition may be prepared by electrospinning a sol-gel.

An exemplary sol-gel is prepared with a silicon alkoxide reagent, such as tetraethyl ortho silicate (TEOS), alcohol solvent, and an acid catalyst. In various embodiments, the sol is transitioned for at least two days (and, in various embodiments, less than seven days) under conditions where humidity and temperature are controlled. The sol-gel is electrospun to create a silica fiber mat with superior texture and properties, which may find use as or in a hemostatic device.

Known processes do not yield a silica fiber composition with sufficient flexibility for many applications, including for wound care or health care applications. Instead, these structures are comparatively brittle, rigid, and compact; mats will easily fracture or break; fiber layers are difficult to separate; and generally lack the physical characteristics for use in health care applications. In various embodiments, to achieve a superior material for tissue repair, it is important to electrospin the sol-gel once it is appropriately ripened (or "transitioned"), to achieve a composition with the desired physical characteristics. By transitioning the sol under controlled environmental conditions, and/or monitoring the preparation of the sol-gel during the ripening process, the relatively short window to successfully electrospin the sol-gel may be identified. In accordance with embodiments of the invention, the composition is non-rigid and has a soft texture similar to that of cotton.

The fibers may have a variable diameter, such as in the range of from about 50 nm to 5 µm. In some embodiments, the fibers are predominately in the range of about 100 nm to about 2 µm, or predominately in the range of about 200 to about 800 nm.

In some embodiments, the sol-gel for preparing the silica fiber composition is prepared by a method that includes preparing a first mixture containing an alcohol solvent, a silicon alkoxide reagent such as tetraethyl ortho silicate (TEOS); preparing a second mixture containing an alcohol solvent, water, and an acid catalyst; fully titrating the second mixture into the first mixture; and processing (ripening) the combined mixture under controlled environmental conditions to form a gel for electrospinning.

In some embodiments, the silicon alkoxide reagent is TEOS. Alternative silicon alkoxide reagents include those with the formula Si(OR)₄, where R is from 1 to 6, and preferably 1, 2, or 3. In some embodiments, the alcohol solvent is an anhydrous denatured ethanol, or in some embodiments, methanol, propanol, butanol or any other suitable alcohol solvent. The first mixture may be agitated, for example, using a magnetic stirrer or similar agitation means. The second mixture contains an alcohol solvent, water, and an acid catalyst. The alcohol solvent in the second mixture may also be an anhydrous denatured alcohol, or may be methanol, propanol, butanol or any other suitably provided alcohol solvent. Water may be distilled water or deionized water. Enough acid catalyst is added to the mixture to aid in the reaction. This acid catalyst may be hydrochloric acid, or may be sulfuric acid or other suitable acid catalyst. The second mixture may be agitated, for example, with a magnetic stirrer or other agitation means. In some embodiments, the first mixture (or sol) and the second mixture (or sol) are created without the use of direct heat.

In some embodiments, the sol will contain about 70% to about 90% by weight silicon alkoxide (e.g., TEOS), about 5% to about 25% by weight alcohol solvent (e.g., anhydrous ethanol), an acid catalyst (e.g., less than about 0.1% by weight when using HCl) and the balance water.

In some embodiments, the sol contains 70% to 90% tetraethyl orthosilicate (TEOS) by weight, 8% to 25% ethanol by weight, 1% to 10% water by weight, and an acid catalyst. In some embodiments, the sol contains 75% to 85% by weight TEOS, 12% to 20% by weight ethanol, and about 2% to 5% by weight water. An exemplary sol contains about 80% by weight TEOS, about 17% by weight ethanol, and about 3% by weight water. In some embodiments, the acid catalyst is HCl. For example, the sol may contain less than about 0.1% HCl by weight. For example, the sol may contain from 0.02% to 0.08% HCl by weight. In various embodiments, the sol does not contain an organic polymer, or other substantial reagents, such that the fiber composition will be substantially pure SiO₂. In various embodiments, the sol does not include inorganic salts (e.g., sodium chloride, lithium chloride, potassium chloride, magnesium chloride, calcium chloride, and/or barium chloride), nor are, in various embodiments, inorganic salts mixed with other components of the sol or into the sol itself. In various embodiments, the fiber composition (and the sol) does not include metals or metal oxides (e.g., TiO₂ or ZrO₂). In various embodiments, the fiber composition consists essentially of SiO₂, i.e., contains only SiO₂ and unintentional impurities, and, in some embodiments, species and/or complexes resulting from the incomplete conversion of the sol to SiO₂ (e.g., water and/or chemical groups such as ethoxy groups, silanol groups, hydroxyl groups, etc.).

According to various embodiments, the first mixture and the second mixture are combined by dripping or titrating the second mixture into the first mixture, preferably with agitation. The combined mixture is then further processed by allowing the sol to ripen in a controlled environment until a substantial portion of the alcohol solvent has evaporated to create a sol-gel suitable for electrospinning.

In various embodiments, the sol is not exposed to heat over 65.6°C (150°F) or heat over 37.8°C (100°F), so as to avoid accelerating the transition. The controlled environment may include an enclosure with at least one vent and optionally an exhaust fan to draw gases away from the mixture. The enclosure may involve controlled conditions in terms of humidity, temperature, and optionally barometric pressure. For example, the humidity may be controlled (e.g., via use of conventional humidifiers and/or dehumidifiers) within the range of about 30% to about 90%, such as from about 40% to about 80%, or in some embodiments, from about 50% to about 80%, or from about 50% to about 70% (e.g., about 55%, or about 60%, or about 65%). Some humidity may be helpful to slow evaporation of solvent, and thereby lengthen the window for successful electrospinning. In some embodiments, the temperature is in the range of from about 10°C (50°F) to about 32.2°C (90°F) such as from about 15.6°C (60°F) about 26.7°C (80°F), or from about 18.3°C (65°F) to about 23.9°C (75°F). In some embodiments, barometric pressure is optionally controlled (e.g., using a low pressure vacuum source such as a pump or a fan). By controlling the environmental conditions during ripening, the gel can be electrospun during the time when spinning is optimal, which may occur in a small window of only several minutes if the ripening process is too accelerated, such as with direct heat. When ripening the sol at a constant humidity of about 55% and temperature of about 22.2°C (72°F), the sol will ripen (gelatinize) in a few days, and the window for successful electrospinning may be expanded to at least several hours, and in some embodiments several days. In various embodiments, the ripening process takes at least 2 days, or at least 3 days in some embodiments. However, in various embodiments the ripening does not take more than 10 days, or more than 7 days. In some embodiments, the ripening process takes from 2 to 7 days or from 2 to 5 days or from 2 to 4 days (e.g., about 2, about 3, or about 4 days). In various embodiments, the sol-gel is spinnable well before it transitions into a more solidified, non-flowable mass.

The enclosure space for ripening the sol-gel may include a vent on at least one surface for exhausting gases from within the enclosure, and optionally the vent may include a fan for exhausting gases produced during the ripening process. The enclosure space may optionally include a heating source for providing a nominal amount of heat within the enclosure space, to maintain a preferred temperature. In some embodiments, a source of humidity (e.g., an open container of water or other aqueous, water-based liquid) is provided within the enclosure environment to adjust the humidity to a desired range or value. The enclosure may further include one or more environmental monitors, such as a temperature reading device (e.g., a thermometer, thermocouple, or other temperature sensor) and/or a humidity reading device (e.g., a hygrometer or other humidity sensor).

In some embodiments, the sol-gel is electrospun after a ripening process of at least 2 days, or at least 36 hours, or at least 3 days, or at least 4 days, or at least 5 days at the controlled environmental conditions (but in various embodiments, not more than 10 days or not more than 7 days under the controlled environmental conditions). By slowing the ripening process, the ideal time to spin the fibers may be identified. The weight of the sol-gel may be used as an indicator of when the sol-gel is at or near the ideal time to electrospin. Without intending to be bound by theory, it is believed that the viscosity of the sol-gel is a poor determinant for identifying the optimal time for electrospinning. For example, in various embodiments, the sol-gel is from about 10% to about 60% of the original weight of the sol (based on loss of alcohol solvent during transitioning). In some embodiments, the sol-gel is from 15 to 50% of the original weight of the sol, or in the range of about 20 to about 40% of the original weight of the sol.

In some embodiments, the sol-gel is ripened for at least 2 days, or at least 36 hours, or at least 3 days, or at least 4 days, or at least 5 days, and is electrospun when the ethylene vapors produced by the composition are between about 10% and about 40% of the vapors produced by the starting sol, such as in the range of about 10% and about 25%, such as in the range of about 10 to about 20%. Ethylene is a colorless flammable gas with a faint sweet and musky odor (which is clearly evident as solvent evaporation slows). Ethylene is produced by the reaction of ethanol and acid. Ethylene may optionally be monitored in the vapors using a conventional ethylene monitor. In other embodiments, gases produced by the sol during the sol ripening process are monitored to determine the suitable or optimal time for electrospinning. Gas profiles may be monitored using gas chromatography.

The processing of the sol-gel mixture may require stirring or other agitation of the mixtures at various intervals or continuously due to the development of silicone dioxide crystalline material on the top surface of the mixtures. This development of crystalline material on the top surface slows the processing time and it is believed that the crystalline material seals off exposure of the mixture to the gaseous vacuum provided within the enclosure space. In some embodiments, any solid crystalline material is removed from the mixture.

Upon completion of the sol-gel process, the sol-gel is then electrospun using any known technique. The sol or sol-gel may be preserved (e.g., frozen or refrigerated) if needed (and such time generally will not apply to the time for ripening). An exemplary process for electrospinning the sol-gel is described in Choi, Sung-Seen, et al., Silica nanofibers from electrospinning/sol-gel process, Journal of Materials Science Letters 22, 2003, 891-893. Exemplary processes for electrospinning are further disclosed in U.S. Patent No. 8,088,965.

In an exemplary electrospinning technique, the sol-gel is placed into one or more syringe pumps that are fluidly coupled to one or more spinnerets. The spinnerets are connected to a high-voltage (e.g., 5 kV to 50 kV) source and are external to and face toward a grounded collector drum. The drum rotates during spinning, typically along an axis of rotation approximately perpendicular to the spinning direction extending from the spinnerets to the drum. As the sol-gel is supplied to the spinnerets from the syringe pumps (or other holding tank), the high voltage between the spinnerets and the drum forms charged liquid jets that are deposited on the drum as small entangled fibers. As the drum rotates and electrospinning continues, a fibrous mat of silica fibers is formed around the circumference of the drum. In various embodiments, the spinnerets and syringe pump(s) may be disposed on a movable platform that is movable parallel to the length of the drum. In this manner, the length along the drum of the resulting fiber mat may be increased without increasing the number of spinnerets. The diameter of the drum may also be increased to increase the areal size of the electrospun mat. The thickness of the mat may be largely dependent upon the amount of sol-gel used for spinning and thus the amount of electrospinning time. For example, the mat may have a thickness of greater than about 0.32 cm (1/8 inch), or greater than about 0.64 cm (1/4 inch), or greater than about 0.85 cm (1/3 inch), or greater than about 1.27cm (1/2 inch).

Silica fiber mats and compositions produced in accordance with embodiments of the present invention exhibit one or more beneficial properties when compared to fiber compositions spun at non-optimal times (e.g., with inadequate ripening of the sol-gel). For example, fiber mats and compositions in accordance with embodiments of the invention do not burn, char, or visibly degrade upon direct application of heat or open flame. In contrast, various fiber compositions spun at non-optimal times will exhibit charring and/or visible color change when exposed to sufficient heat or open flame. Moreover, fiber mats and compositions in accordance with embodiments of the invention effectively wick moisture (e.g., water or bodily fluids), absorbing such fluid into the fiber mat. In contrast, various fiber compositions spun at non-optimal times will not visibly absorb or wick moisture even when directly applied thereto; such compositions tend to be hydrophobic. Finally, fiber mats and compositions in accordance with embodiments of the invention are fluffy and may be easily shaped to uneven, non-uniform, and/or nonplanar (e.g., curved) surfaces or shapes without fracturing or loss of structural integrity; thus, such compositions may be readily applied to or conformed to a variety of different surfaces. In contrast, various fiber compositions spun at non-optimal times tend to be flat, plate-like, brittle, and will at least partially fracture if excessively mechanically shaped or bent.

Fiber layers may be easily separated from the mat for, e.g., application to hemorrhaging tissue. In some embodiments, the composition is electrospun with a thickness of from about 0.32 cm (1/8 inch) to about 1.27 cm (1/2 inch). For example, the composition may be electrospun with a thickness of greater than about 0.32 cm (1/8 inch). or greater than about 0.64 cm (1/4 inch) thick, or about 0.64 cm (1/4 inch) thick in some embodiments.

The material is able to hold many times its weight in water, typically more than about 50 times or about 70 times its weight in water. This feature allows the material to absorb a large amount of blood, while being impenetrable by blood cells, thereby quickly forming a physical barrier. In various embodiments, the material enhances the coagulation cascade when exposed to blood. The material may be used as fiber mats that are folded and/or clumped to prepare a suitable mass of material for packing a hemorrhaging site, including a hemorrhage in a body cavity. In some embodiments, the silica fiber material is processed into a flocculent material. A flocculent material may be loosely clumped, and may be created by shredding and/or clumping portions of the electrospun fiber mats. The flocculent material may be used to pack the hemorrhaging site. Alternatively, the silica fiber material may be applied as a powder or dust, gel, paste, or as an additive to a flowable base material. Bulking agents may be added to the material, and may include various synthetic and natural polymers, and may include materials such as poloxamers, polyethylene glycol, polyvinyl alcohol, collagen, gelatin, cellulose, chitosan, and fibrin, among others.

In various embodiments, the silica fiber composition may then be divided into small fibrous fragments that may be applied to the hemorrhage. The resulting fibrous fragments are each intertwined collections of silica fibers, rather than unitary solid particles. In some embodiments, the electrospun mat may be fractured, cut, ground, milled, or otherwise divided into small fragments that maintain a fibrous structure. In some embodiments, the mat (or one or more portions thereof) is rubbed through one or more screens or sieves, and the mesh size of the screen determines, at least in part, the size of the resulting fibrous fragments produced from the electrospun mat. For example, the mat or mat portions may be rubbed through a succession of two or more screens having decreasing mesh sizes (e.g., screens having mesh numbers of 100, 200, 300, or even 400), in order to produce a collection of fibrous fragments having the desired sizes.

As used herein, the term "fibrous fragments" (or "fibrous-mat fragments," or simply "fragments") refers to small dust-like particles, parts, or flakes of a fibrous mat having an average dimension larger (e.g., 5×, 10×, or even 100×) than the width of at least some of the fibers of the mat. In various embodiments, the average size of a fibrous fragment is in the range of approximately 20 µm to approximately 200 µm along the longest axis. Fibrous fragments may thus resemble microscopic-scale versions of the electrospun mat itself, e.g., intertwined collections of silica fibers, and thus typically are porous. Thus, fibrous fragments may be contrasted with other types of micro-scale particles, such as the substantially spherical particles used in colloidal silica, which are each unitary, individual units or grains, rather than small collections of fibers.

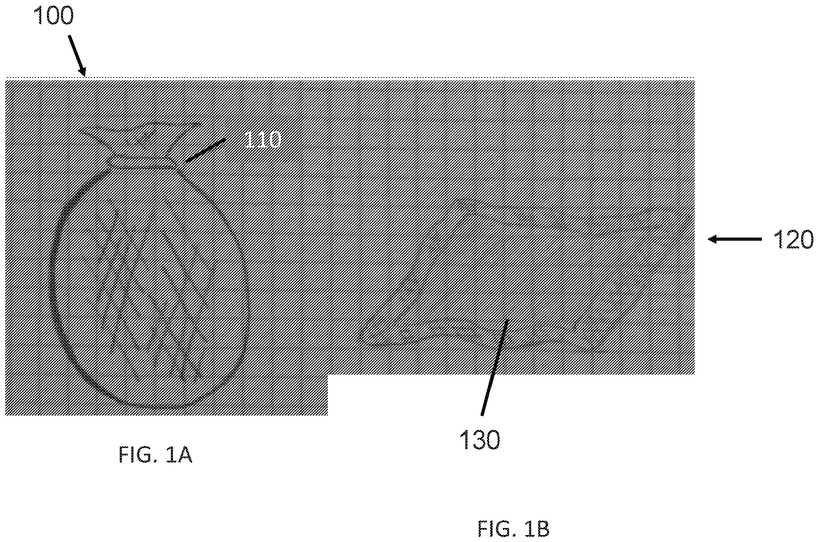

In some embodiments, the silica fibers are provided with a material to facilitate handling and application to the wound site. For example, the silica fiber material may be combined with a backing material (which may be gauze or other fabric in some embodiments), or inside a flexible container (e.g., a bag) made of any material that allows the silica fiber material to be emptied or placed at the site of hemorrhage. FIGS. 1A and 1B depict examples in which the silica fiber material is provided within a flexible container or on a flexible backing material for application to a wound site. For example, FIG. 1A shows a flexible bag 100 containing the silica fiber material and closed via use of a tie 110. In various embodiments, the bag 100 includes, consists essentially of, or consists of a gauze or similar material, e.g., combat gauze. FIG. 1B depicts another embodiment, in which a pad or backing 120 contains silica fiber material 130 therein and/or thereon. In various embodiments, the pad or backing 120 includes, consists essentially of, or consists of a gauze or similar material, e.g., combat gauze. In various embodiments, all or a portion of the periphery of the pad or backing 120 may be adhesive to help keep the pad or backing 120 in place after application.

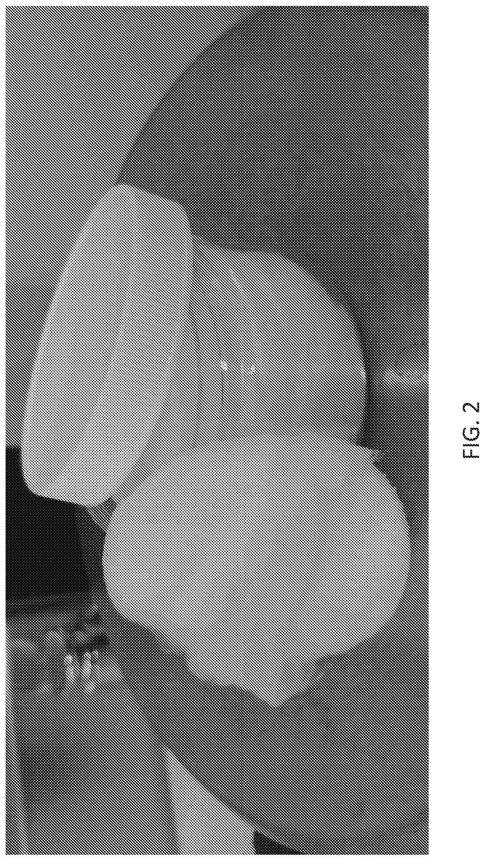

In some embodiments, the silica fiber material itself is applied to the wound site without use of a backing material or container. FIG. 2 depicts an example embodiment in which a silica fiber pad is shaped as a disc for application to a wound site. As shown, such pads may be stacked and/or stored in an appropriate container, such as the jar depicted in FIG. 2.

In some embodiments, the fiber composition is applied directly to the site of hemorrhage by packing the site with the material, in any form, and applying pressure where and as possible. The fiber composition will absorb the blood and large amounts of fluid, although the blood cells cannot penetrate the material, and will quickly form a physical barrier for further loss of blood. In some embodiments, even for severe hemorrhage (e.g., grade 3 or 4 hemorrhage), the silica fiber material may stop blood flow within about 60 seconds, or in some embodiments, within about 30 seconds, or in some embodiments within about 15 seconds. By quickly stopping a severe hemorrhage at the site of injury, the patient may more quickly and safely be transported to a surgical facility. The silica fiber material, or at least a portion thereof, may be easily removed by a physician during surgery.

In some embodiments, the rate of blood flow at the site of the hemorrhage is sufficient to render direct application of the fiber composition difficult. For example, blood flow from a ruptured blood vessel may resist or prevent contact between the silica fiber material and the damaged vessel. Thus, in various embodiments the fiber composition is applied directly to the site using a delivery device that simultaneously delivers the fiber composition to the wound site while aspirating excess blood at or near the wound site. In this manner, direct contact between the fiber composition and the wound site is facilitated.

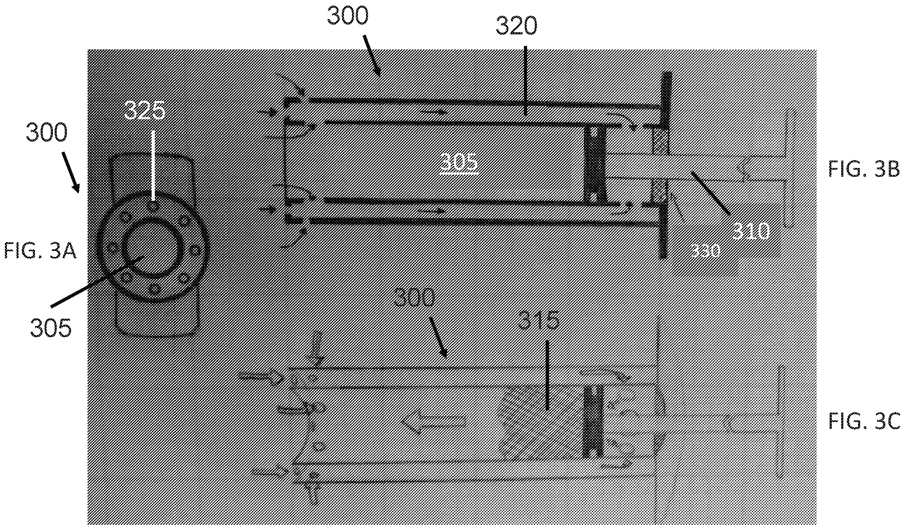

FIGS. 3A-3C schematically depict an exemplary delivery device 300 for fiber compositions in accordance with embodiments of the present invention. As shown, the delivery device 300 may include a hollow central bore (or "chamber") 305 that is open at one end. The opposing end may be occluded by a movable plunger 310. The fiber composition 315 may be packed or otherwise placed within the central bore 305, as shown in FIG. 3C, and actuation of the plunger 310 by the user forces the fiber composition 315 out of the central chamber 305 and onto (or at least near) the wound site. As shown in FIGS. 3B and 3C, as the plunger 310 is actuated, the central bore 305 is effectively divided into two separate compartments, a lower compartment from which the fiber composition 315 emerges into the wound site, and an upper compartment separated from the lower compartment by a portion of the plunger 310.

In order to facilitate sufficient contact between the fiber composition and a bleeding wound without the fiber composition being drawn away from the wound by the flow of blood, in various embodiments the delivery device 300 aspirates away excess blood simultaneously with the delivery of the fiber composition. As shown in FIGS. 3B and 3C, in various embodiments the delivery device 300 may include an outer annular compartment 320 that is coaxial with the hollow central bore 305. As shown, the outer compartment 320 defines openings 325 that facilitate a fluid connection between the wound site and at least the upper compartment of the central bore 305. As indicated in FIGS. 3B and 3C, as the plunger 310 is actuated and the fiber composition is delivered to the wound site, the action of the plunger 310 creates suction in the upper compartment of the central bore 305. As a result, excess blood from the wound site (e.g., from one or more hemorrhaging blood vessels) is drawn into the outer annular compartment 320 of the delivery device 300 and into the upper compartment. In this manner, excess blood or other fluid may be removed from the wound site while the fiber composition is simultaneously applied to the wound site, facilitating direct contact between the fiber composition and the hemorrhage to be treated.

As shown in FIGS. 3B and 3C, in some embodiments the outer annular compartment 320 is also fluidly connected to the lower compartment of the central bore 305 by one or more openings. In this manner, excess blood entering the lower compartment during application of the fiber composition may also be drawn into the upper compartment.

In various embodiments, the openings fluidly connecting the outer annular compartment 320 with the wound site and/or with the upper compartment are sufficiently small such that little or none of the fiber composition is inadvertently aspirated away from the wound site. Alternatively or in addition, the openings may include filters or screens thereon that prevent the flow of the fiber composition (at least larger pieces thereof) or other solids from being aspirated into the outer annular compartment and/or the upper compartment of the delivery device. As also shown in FIG. 3B, the upper compartment of the central bore may be sealed with an annular gasket 330 that permits movement of the plunger 310 while reducing or substantially eliminating entry of solid or liquid materials into the upper compartment of the central bore 305.

In various embodiments, all or a portion of the delivery device 300 includes, consists essentially of, or consists of one or more biocompatible materials, e.g., one or more plastics, other polymeric materials, or stainless steel.

The silica fiber material may be used for human and animal treatment. In some embodiments, the subject is a mammal. Subjects include veterinary patients such as a dog, cat, pig, or horse, among others. In some embodiments, the patient is a human patient.

The World Health Organization includes grades of hemorrhage from 0 to 5. In some embodiments, the hemorrhage is at least a Grade 2 hemorrhage (e.g., a Grade 3 or Grade 4 hemorrhage). A Grade 2 hemorrhage is mild but clinically significant. In some embodiments, the hemorrhage is a Grade 4 hemorrhage. A Grade 4 hemorrhage is defined as severe bleeding that would typically require transfusion. A Grade 5 hemorrhage is associated with fatality. In some embodiments, the hemorrhage is an arterial hemorrhage. Arterial hemorrhage often exhibits spurting as a jet which rises and falls in time with the pulse. In protracted bleeding, and when quantities of intravenous fluids other than blood are given, it can become watery in appearance. In some embodiments, the hemorrhage is a venous hemorrhage. Venous hemorrhage is a darker red, a steady and copious flow. Blood loss is particularly rapid when large veins are opened.

In some embodiments, the hemorrhage is bleeding during or after surgery. For example, the hemorrhage may be an organ bleed. In these embodiments, the silica fiber material may be applied directly to the bleed, using portions of silica fiber mats, or various other forms, such as powder or dusts, pastes or gels, or a flowable base comprising silica fiber dust.

In some embodiments, the hemorrhage is a primary hemorrhage, which occurs at the time of injury or operation. In some embodiments, the hemorrhage is a reactionary hemorrhage. Reactionary hemorrhage may follow primary hemorrhage within 24 hours (usually 4 to 6 hours) and is mainly due to rolling ('slipping') of a ligature, dislodgement of a clot or cessation of reflex vasospasm. In some embodiments, the hemorrhage is a secondary hemorrhage. Secondary hemorrhage occurs after 7 to 14 days, and is due to infection and sloughing.

In some embodiments, the hemorrhage is a traumatic injury bleed. For example, the hemorrhage is a non-compressible hemorrhage. In some embodiments, the hemorrhage is a cavity bleed. In some embodiments, the injury is a crushing injury. In some embodiments, the injury is a gunshot injury or explosion injury.

In some embodiments, the hemorrhage is epistaxis, and the silica fiber material packed inside the nasal cavity.

In some embodiments, the hemorrhage is external. For example, the silica fiber material may be shaped as pads or bandages, or used with various types of conventional bandage materials (e.g., cotton gauze) and wound dressings to encourage bleeding control.

In some embodiments, the patient is on therapy inhibiting the coagulation pathway. These therapies can include heparin (unfractionated or LMWH). Alternatively, the patient may be on therapy with a Factor Xa inhibitor or direct thrombin inhibitor, or other direct inhibitor of the coagulation pathway. In some embodiments, the patient has a genetic coagulation disorder, such as hemophilia, thrombocytopenia, low platelet count, or von Willebrand disease.

Other aspects and embodiments of the invention will be apparent from the following non-limiting examples.

### Examples

### Example 1: Preparation of silica fiber composition

SiO₂ fibers were prepared using an electrical spinning process, where a sol-gel is spun onto a roller system creating a sheet. The sol-gel is made in two parts. First, TEOS is mixed with ethanol, and then a second mixture containing HCl, water, and ethanol is titrated into the mixture. The sol-gel is then allowed to ripen for a few days under controlled conditions before spinning.

In one example, the first sol was made by weighing out 384 grams of (TEOS) tetraethyl orthosilicate 98% and 41.8 grams of anhydrous denatured ethanol, and pouring together. The first sol was allowed to let stand in a beaker and a magnetic stirrer was used to create a homogenous solution. The second sol was made by weighing 41.8 grams of anhydrous denatured ethanol, 16.4 grams of distilled water, and 0.34 grams of hydrochloric acid, which was then poured together and mixed for 8 seconds with a magnetic stirrer until a homogenous second sol was formed.

The second sol was then poured into the titration device, which was placed above a beaker containing the first sol . The titration device then dripped about 5 drops per second until a third sol was formed mixing the first sol and the second sol. During the dripping process, the first sol continues to be mixed with a magnetic stirrer while the second sol is dripped into the first sol.

The combined third sol was then placed into an enclosure box. A low pressure vacuum is provided by a fan on medium speed to remove fumes. In the experiment, the air temperature within the box was 27.8°C (82°F) with 60% humidity. In the experiment, the third sol was allowed to sit and process for about three (3) days. By ripening the sol-gel slowly over several days, the sol-gel will transition slowly such that the ideal time to electrospin can be identified.

The mixtures were agitated daily to reduce the build-up of crystalline structures. The third sol begins to transition to sol-gel with evaporation of the alcohol solvent. Sol-gel may be monitored to determine an approximate amount of C₂H₄ (ethylene) in the vapors, which can be in the range of about 10-20% relative to the original sol before ripening. Upon proper gelatinization, the sol-gel is loaded into the electrospinning machine or is frozen to preserve for electrospinning. Proper gelatinization occurs when the total mass of the sol-gel was between about 100 grams and about 180 grams.

The above example can be scaled appropriately to produce desirable structures. To further identify the ideal time to electropsin, portions of the gel can be dripped into the electric field to evaluate the properties of the resulting fibers.

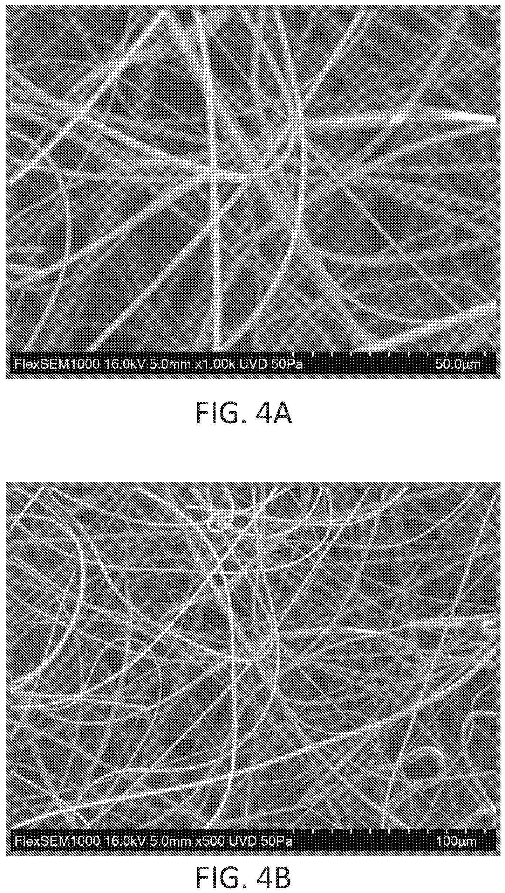

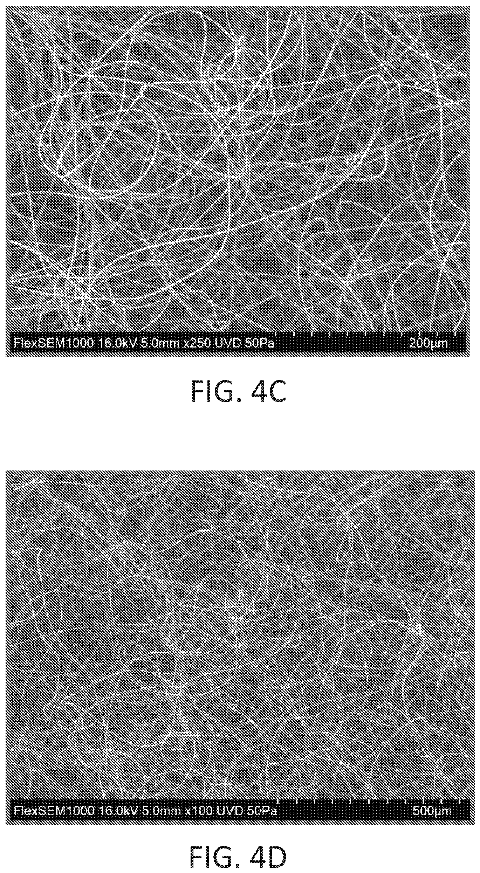

FIGS. 4A-4D are scanning electron microscopy (SEM) images of fibers spun in accordance with this disclosure (50, 100, 200, and 500 micron scales shown). As shown, the fibers are flexible, smooth, dense, and continuous (not fractured). Material with these properties is ideal for treating wounds and animal tissues (e.g., as a collagen mimetic).

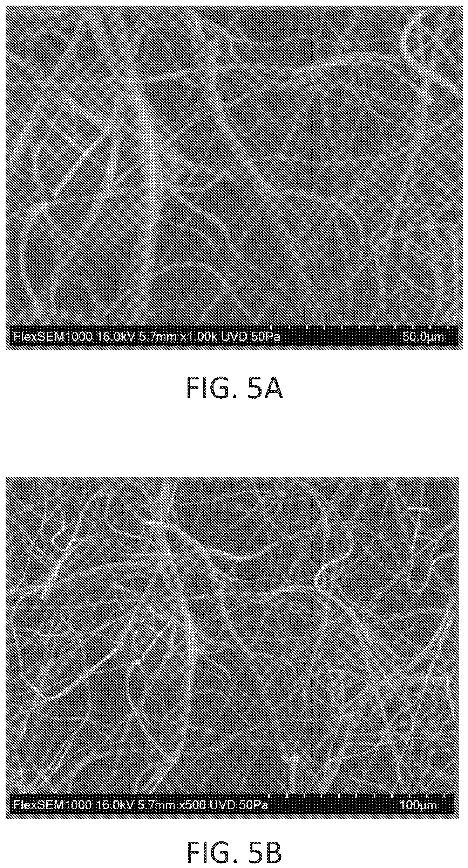

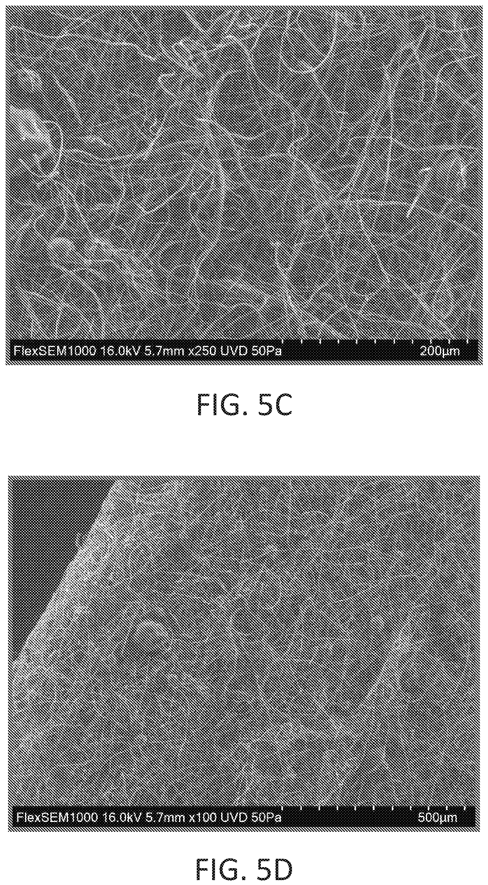

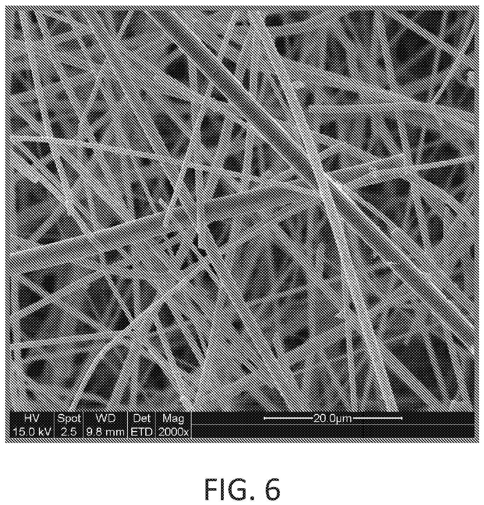

FIGS. 5A-5D are SEM images of fibers that were electrospun at a non-optimal time (before the sol-gel was fully ripened) (50, 100, 200, and 500 micron scale shown). The fibers appear rigid, with many fractures visible, and with formation of clumps. FIG. 6 shows an SEM image (20 micron scale is shown) of fibers from a similar material, where the fibers are clearly rigid with many fractures clearly evident.

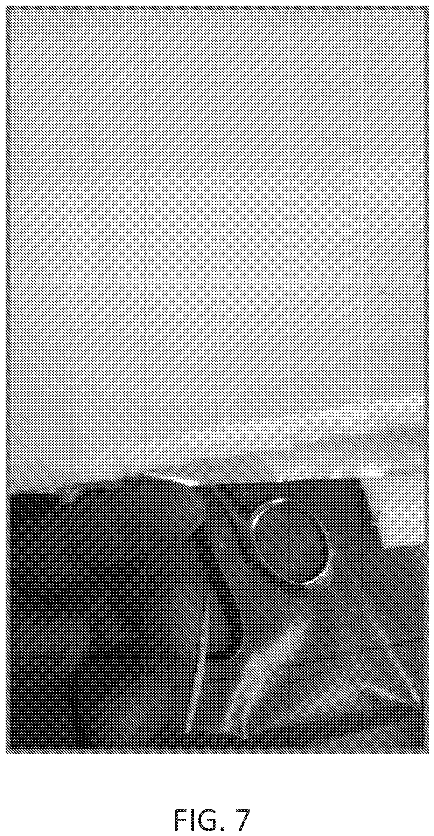

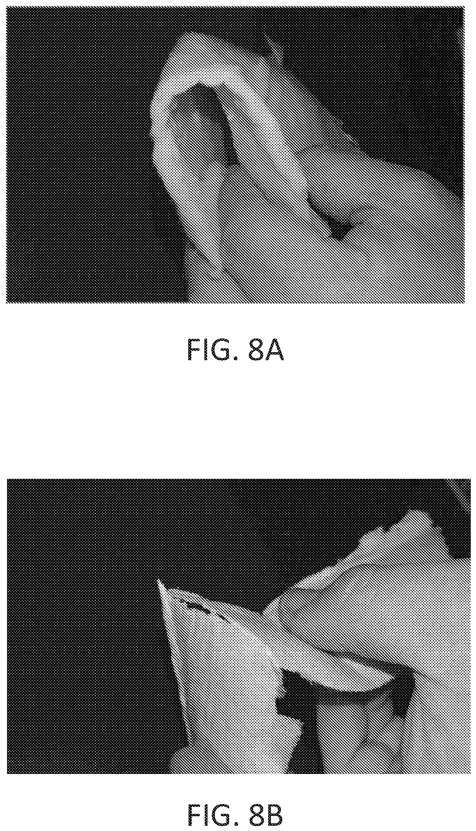

FIG. 7 shows a fiber mat spun in accordance with the disclosure. The flexibility and continuity of the fibers allows mats to be spun at a thickness of 1/4 inch or more. The mat has a soft, flexible texture, and allows for layers of fibers to be easily separated for covering a wound bed. FIGS. 8A and 8B compare a silica fiber mat that was electrospun when the sol-gel was ripened in accordance with this disclosure (FIG. 8A) to a fiber mat that was spun too early, before the sol-gel was optimally ripened (FIG. 8B). The material in FIG. 8A has a soft texture, is very flexible, and can be spun at a thickness that is easily handled for application of fiber layers to a wound site. The material in FIG. 8B is brittle, inflexible, thin, and layers of fiber cannot be easily separated for packing a site of hemorrhage.

### Example 2: Hemostatis study

A swine model was utilized to study the effect of silica fiber compositions in accordance with embodiments of the invention on the hemostasis of lethal extremity arterial hemorrhage. Specifically, muscle tissue was removed to expose the femoral artery, and a biopunch used to sever the artery. After bleeding for several seconds, silica fiber material was applied with limited pressure to the severed vessel, which quickly stopped the active bleed. The subject was kept alive for a period of 3 hours during which the leg was moved back and forth 10 times and then checked for bleeding. The subject was then moved to a sled and dragged 50 yards to simulate extraction of a soldier in combat. The wound was then checked for bleeding. After this, the subject was euthanized, and the injured vessel was excised for analysis. Control subjects were treated with conventional QuikClot Combat Gauze (i.e., a soft nonwoven gauze impregnated with kaolin for acceleration of clotting) for comparison.

In the experiment, the average blood loss and bleeding time for the subjects treated with the silica fiber composition was decreased by approximately a factor of two compared to the control subjects. The final mean arterial pressure and heart rate were also slightly higher for the subjects treated with the silica fiber composition. The body temperature of the subjects treated with the silica fiber composition also trended lower (i.e., improved) during the duration of the study.

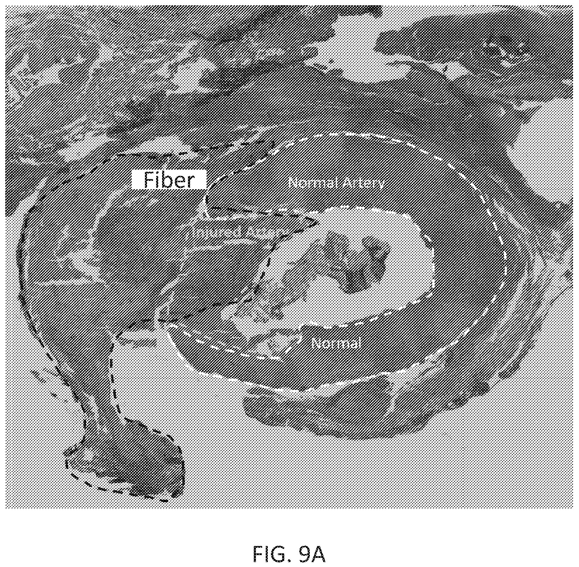

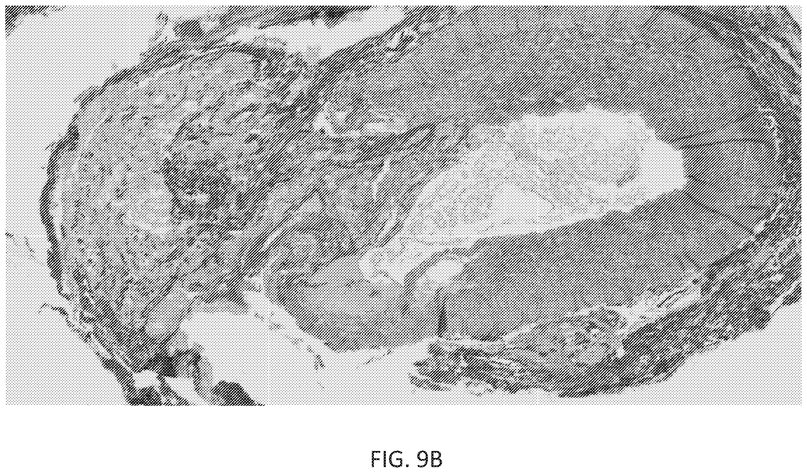

After the experiment, histology was performed utilizing hematoxylin and eosin staining, as shown in FIG. 9A. As observed in the image, the silica fiber composition (designated on the image as "Fiber") occluded the arterial injury (i.e., the outlined portion designated as "Injured Artery"), and there are clotting fibrils and aligned red blood cells outside of the injury site. FIG. 9B shows a Martius-scarlet-blue (MSB) trichrome fibrin stain image of the injury site treated with the silica fiber composition. The image shows the creation of a blood clot around and within the silica fiber sealing the injury site. Cells growing into the silica fiber are similar to those of the vessel wall. Fibrin fibers are intertwined with the silica fibers, and a clear fibrin bridge (indicated by the darker stained fibrils within the injury site) has formed across the edge of the entire injury site where the silica fibers were applied. This is unexpected for such a short treatment time of such a massive injury to a major artery, demonstrating efficacy of embodiments of the present invention for the control and treatment of hemorrhages.

Overall, the silica fiber composition stopped the arterial bleeding much more quickly than the gauze of the control samples. Pressure could be removed, and lack of bleeding observed, even before the end of the three-minute treatment period. When the porcine leg was moved, there was no further bleeding. Hemostasis was maintained, even during manual attempts to dislodge the clot. In contrast, use of the control gauze required more significant pressure to be applied, and there was increased bleeding around the gauze for most of the three-minute treatment period. Unlike the silica fiber composition, the gauze is also designed for removal after hemostasis, and it is therefore not designed to remain within the wound to direct regeneration. The silica fiber composition reduces the time to hemostasis and the amount of total bleeding. It also reduces the number of re-bleeding events, and facilitates tissue coverage and regeneration, ultimately improving the chances of survival of a major bleed.

## Claims

1. Electrospun silica fiber composition for use in treating hemorrhage comprising applying the electrospun silica fiber composition to said hemorrhage in an amount and under conditions sufficient to stop the hemorrhage, wherein the silica fiber composition is prepared by electrospinning a sol that is prepared with 70% to 90% tetraethyl orthosilicate (TEOS) by weight, 8% to 25% ethanol by weight, an acid catalyst, preferably HCl, and the balance water.

2. The electrospun silica fiber composition for use of claim 1, wherein the sol is transitioned for 2 to 7 days under conditions where humidity is within the range of 40% to 80%, and the temperature is within the range of 10 to 32.2 °C (50 to 90° F), preferably, wherein the sol is not exposed to heat over 65.6 °C (150° F) or heat over 37.8 °C (100° F).

3. The electrospun silica fiber composition for use of claim 1, wherein the silica fiber composition is applied as a flocculent material, or alternatively, as a powder, dust, gel, paste or additive to a flowable base material.

4. The electrospun silica fiber composition for use of claim 1 in the treatment of a mammal, preferably a human.

5. The electrospun silica fiber composition for use of any one of claims 1 to 4, wherein the hemorrhage is at least a Grade 2 hemorrhage, preferably, the hemorrhage is a Grade 3 or Grade 4 hemorrhage.

6. The electrospun silica fiber composition for use of claim 5, wherein the hemorrhage is an arterial hemorrhage, or alternatively, the hemorrhage is a venous hemorrhage.

7. The electrospun silica fiber composition for use of claim 5, wherein the hemorrhage is bleeding during or after surgery, preferably the hemorrhage is an organ bleed.

8. The electrospun silica fiber composition for use of any one of claims 5 to 7, wherein the hemorrhage is a primary hemorrhage, or alternatively, wherein the hemorrhage is a reactionary hemorrhage, or alternatively, the hemorrhage is a secondary hemorrhage.

9. The electrospun silica fiber composition for use of any one of claims 1 to 8, wherein the hemorrhage is a traumatic injury bleed, preferably, the hemorrhage is a non-compressible hemorrhage or a cavity bleed.

10. The electrospun silica fiber composition for use of claim 9, wherein the injury is a crushing injury, or alternatively, the injury is a gunshot injury or an explosion injury.

11. The electrospun silica fiber composition for use of claim 5, wherein the hemorrhage is epistaxis, or alternatively, the hemorrhage is external.

12. The electrospun silica fiber composition for use of any one of claims 1 to 11 in a patient being on therapy inhibiting the coagulation pathway.

13. The electrospun silica fiber composition for use of any one of claims 1 to 11, in a patient having a coagulation disorder, wherein, preferably, the patient has hemophilia, thrombocytopenia, low platelet count, or von Willebrand disease.

14. The electrospun silica fiber composition for use of any one of claims 1 to 13, wherein the silica fiber composition is applied to said hemorrhage via a delivery device comprising:
a hollow central bore having an upper end and an open lower end;
disposed at the upper end of the central bore, a movable plunger actuatable by a user of the delivery device to urge the silica fiber composition from the open lower end of the central bore, a portion of the plunger separating the central bore into a lower compartment and an upper compartment; and
an annular outer compartment coaxial with the central bore, fluidly connected to the upper compartment of the central bore, and defining one or more openings for fluid connection with an area proximate the hemorrhage,
wherein, when the plunger is actuated to urge the silica fiber composition from the open lower end of the central bore to the hemorrhage, fluid proximate the hemorrhage is simultaneously aspirated into the outer compartment and thence into the upper compartment of the central bore.

15. The electrospun silica fiber composition for use of claim 14, wherein the central bore defines one or more openings therethrough fluidly connecting the lower compartment with the annular outer compartment.

## Patentansprüche

1. Elektrogesponnene Siliziumdioxidfaser-Zusammensetzung zur Verwendung für die Behandlung von Blutungen, umfassend das Aufbringen der elektrogesponnenen Siliziumdioxidfaser-Zusammensetzung auf die Blutung in einer Menge und unter Bedingungen, die ausreichen, um die Blutung zu stoppen, wobei die Siliziumdioxidfaser-Zusammensetzung durch Elektrospinnen eines Sols hergestellt wird, das mit 70 bis 90 Gew.-% Tetraethylorthosilikat (TEOS), 8 bis 25 Gew.-% Ethanol, einem Säurekatalysator, vorzugsweise HCl, und dem Rest Wasser hergestellt wird.

2. Elektrogesponnene Siliziumdioxidfaser-Zusammensetzung zur Verwendung nach Anspruch 1, wobei das Sol 2 bis 7 Tage lang unter Bedingungen überführt wird, bei denen die Feuchtigkeit im Bereich von 40 % bis 80 % liegt und die Temperatur im Bereich von 10 bis 32,2 °C (50 bis 90° F) liegt, wobei das Sol vorzugsweise keiner Hitze über 65,6 °C (150° F) oder Hitze über 37,8 °C (100° F) ausgesetzt wird.

3. Elektrogesponnene Siliziumdioxidfaser-Zusammensetzung zur Verwendung nach Anspruch 1, wobei die Siliziumdioxidfaser-Zusammensetzung als flockiges Material oder alternativ als Pulver, Staub, Gel, Paste oder Zusatz zu einem fließfähigen Basismaterial aufgebracht wird.

4. Elektrogesponnene Siliziumdioxidfaser-Zusammensetzung zur Verwendung nach Anspruch 1 für die Behandlung eines Säugetiers, vorzugsweise eines Menschen.

5. Elektrogesponnene Siliziumdioxidfaser-Zusammensetzung zur Verwendung nach einem der Ansprüche 1 bis 4, wobei die Blutung mindestens eine Blutung des Grades 2 ist, vorzugsweise ist die Blutung eine Blutung des Grades 3 oder 4.

6. Elektrogesponnene Siliziumdioxidfaser-Zusammensetzung zur Verwendung nach Anspruch 5, wobei die Blutung eine arterielle Blutung ist, oder alternativ die Blutung eine venöse Blutung ist.

7. Elektrogesponnene Siliziumdioxidfaser-Zusammensetzung zur Verwendung nach Anspruch 5, wobei die Blutung eine Blutung während oder nach einer Operation ist, vorzugsweise ist die Blutung eine Organblutung.

8. Elektrogesponnene Siliziumdioxidfaser-Zusammensetzung zur Verwendung nach einem der Ansprüche 5 bis 7, wobei die Blutung eine primäre Blutung ist, oder alternativ, wobei die Blutung eine reaktive Blutung ist, oder alternativ, die Blutung eine sekundäre Blutung ist.

9. Elektrogesponnene Siliziumdioxidfaser-Zusammensetzung zur Verwendung nach einem der Ansprüche 1 bis 8, wobei die Blutung eine Blutung aus einer traumatischen Verletzung ist, vorzugsweise ist die Blutung eine nicht komprimierbare Blutung oder eine Hohlraumblutung.

10. Elektrogesponnene Siliziumdioxidfaser-Zusammensetzung zur Verwendung nach Anspruch 9, wobei die Verletzung eine Quetschverletzung ist, oder alternativ, die Verletzung eine Schussverletzung oder eine Explosionsverletzung ist.

11. Elektrogesponnene Siliziumdioxidfaser-Zusammensetzung zur Verwendung nach Anspruch 5, wobei die Blutung eine Epistaxis ist, oder alternativ die Blutung extern ist.

12. Elektrogesponnene Siliziumdioxidfaser-Zusammensetzung zur Verwendung nach einem der Ansprüche 1 bis 11 bei einem Patienten, der eine Therapie zur Hemmung des Gerinnungsweges erhält.

13. Elektrogesponnene Siliziumdioxidfaser-Zusammensetzung zur Verwendung nach einem der Ansprüche 1 bis 11 bei einem Patienten mit einer Gerinnungsstörung, wobei der Patient vorzugsweise an Hämophilie, Thrombozytopenie, niedriger Thrombozytenzahl oder von-Willebrand-Krankheit leidet.

14. Elektrogesponnene Siliziumdioxidfaser-Zusammensetzung zur Verwendung nach einem der Ansprüche 1 bis 13, wobei die Siliziumdioxidfaser-Zusammensetzung auf die Blutung über eine Abgabevorrichtung aufgebracht wird, die Folgendes umfasst:
eine hohle zentrale Bohrung mit einem oberen Ende und einem offenen unteren Ende;
einen am oberen Ende der zentralen Bohrung angeordneten beweglichen Kolben, der von einem Benutzer der Abgabevorrichtung betätigt werden kann, um die Siliziumdioxidfaser-Zusammensetzung aus dem offenen unteren Ende der zentralen Bohrung herauszudrücken, wobei ein Teil des Kolbens die zentrale Bohrung in ein unteres Abteil und ein oberes Abteil trennt; und
ein ringförmiges äußeres Kompartiment, das koaxial mit der zentralen Bohrung ist, mit dem oberen Kompartiment der zentralen Bohrung in Fluidverbindung steht und eine oder mehrere Öffnungen zur Fluidverbindung mit einem Bereich in der Nähe der Blutung definiert,
wobei, wenn der Kolben betätigt wird, um die Siliziumdioxidfaser-Zusammensetzung vom offenen unteren Ende der zentralen Bohrung zur Blutung zu drücken, Flüssigkeit in der Nähe der Blutung gleichzeitig in das äußere Abteil und von dort in das obere Abteil der zentralen Bohrung gesaugt wird.

15. Elektrogesponnene Siliziumdioxid-Faserzusammensetzung zur Verwendung nach Anspruch 14, wobei die zentrale Bohrung eine oder mehrere Öffnungen definiert, die das untere Kompartiment mit dem ringförmigen äußeren Kompartiment fluidmäßig verbinden.

## Revendications

1. Composition de fibres de silice électrofilées pour une utilisation dans le traitement d'une hémorragie comprenant l'application de la composition de fibres de silice électrofilées sur ladite hémorragie en une quantité et dans des conditions suffisantes pour arrêter l'hémorragie, dans laquelle la composition de fibres de silice est préparée par électrofilage d'un sol qui est préparé avec 70 % à 90 % en poids d'orthosilicate de tétraéthyle (TEOS), 8 % à 25 % en poids d'éthanol, un catalyseur acide, de préférence HCl, et le reste étant de l'eau.

2. Composition de fibres de silice électrofilées pour une utilisation selon la revendication 1, dans laquelle le sol est soumis à une transition pendant 2 à 7 jours dans des conditions où l'humidité se situe dans la plage de 40 % à 80 %, et la température se situe dans la plage de 10 à 32,2 °C (50 à 90 °F), de préférence, dans laquelle le sol n'est pas exposé à une chaleur supérieure à 65,6 °C (150 °F) ou à une chaleur supérieure à 37,8 °C (100 °F).

3. Composition de fibres de silice électrofilées pour une utilisation selon la revendication 1, dans laquelle la composition de fibres de silice est appliquée sous forme de matériau floculant, ou en variante, sous forme de poudre, de poussière, de gel, de pâte ou d'additif à un matériau de base fluide.

4. Composition de fibres de silice électrofilées pour une utilisation selon la revendication 1 dans le traitement d'un mammifère, de préférence un humain.

5. Composition de fibres de silice électrofilées pour une utilisation selon l'une des revendications 1 à 4, dans laquelle l'hémorragie est au moins une hémorragie de grade 2, de préférence, l'hémorragie est une hémorragie de grade 3 ou de grade 4.

6. Composition de fibres de silice électrofilées pour une utilisation selon la revendication 5, dans laquelle l'hémorragie est une hémorragie artérielle ou, en variante, l'hémorragie est une hémorragie veineuse.

7. Composition de fibres de silice électrofilées pour une utilisation selon la revendication 5, dans laquelle l'hémorragie est un saignement pendant ou après une intervention chirurgicale, de préférence l'hémorragie est un saignement d'organe.

8. Composition de fibres de silice électrofilées pour une utilisation selon l'une des revendications 5 à 7, dans laquelle l'hémorragie est une hémorragie primaire, ou en variante, dans laquelle l'hémorragie est une hémorragie réactionnelle, ou en variante, l'hémorragie est une hémorragie secondaire.

9. Composition de fibres de silice électrofilées pour une utilisation selon l'une des revendications 1 à 8, dans laquelle l'hémorragie est un saignement de blessure traumatique, de préférence, l'hémorragie est une hémorragie non compressible ou un saignement de cavité.

10. Composition de fibres de silice électrofilées pour une utilisation selon la revendication 9, dans laquelle la blessure est une blessure par écrasement ou, en variante, la blessure est une blessure par balle ou une blessure par explosion.

11. Composition de fibres de silice électrofilées pour une utilisation selon la revendication 5, dans laquelle l'hémorragie est une épistaxis ou, en variante, l'hémorragie est externe.

12. Composition de fibres de silice électrofilées pour une utilisation selon l'une des revendications 1 à 11 chez un patient qui est sous traitement inhibant la voie de coagulation.

13. Composition de fibres de silice électrofilées pour une utilisation selon l'une des revendications 1 à 11, chez un patient présentant un trouble de la coagulation, dans laquelle, de préférence, le patient souffre d'hémophilie, de thrombocytopénie, d'une faible numération plaquettaire ou de la maladie de von Willebrand.

14. Composition de fibres de silice électrofilées pour une utilisation selon l'une des revendications 1 à 13, dans laquelle la composition de fibres de silice est appliquée sur ladite hémorragie par le biais d'un dispositif d'administration comprenant :
un orifice central creux présentant une extrémité supérieure et une extrémité inférieure ouverte ;
disposé au niveau de l'extrémité supérieure de l'orifice central, un piston mobile actionnable par un utilisateur du dispositif d'administration pour pousser la composition de fibres de silice depuis l'extrémité inférieure ouverte de l'orifice central, une partie du piston séparant l'orifice central en un compartiment inférieur et un compartiment supérieur ; et
un compartiment extérieur annulaire coaxial à l'orifice central, relié de manière fluidique au compartiment supérieur de l'orifice central, et définissant une ou plusieurs ouvertures pour une liaison fluidique avec une zone proche de l'hémorragie,
dans laquelle, lorsque le piston est actionné pour pousser la composition de fibres de silice depuis l'extrémité inférieure ouverte de l'orifice central vers l'hémorragie, le fluide proche de l'hémorragie est simultanément aspiré dans le compartiment extérieur et de là dans le compartiment supérieur de l'orifice central.

15. Composition de fibres de silice électrofilées pour une utilisation selon la revendication 14, dans laquelle l'orifice central définit une ou plusieurs ouvertures à travers celui-ci reliant de manière fluidique le compartiment inférieur au compartiment extérieur annulaire.
